(19) Europäisches Patentamt
European Patent Office
Office européen des brevets

(11) EP 2 448 924 B1

(12) # FASCICULE DE BREVET EUROPEEN

(45) Date de publication et mention de la délivrance du brevet:
**04.09.2013 Bulletin 2013/36**

(21) Numéro de dépôt: **10742179.4**

(22) Date de dépôt: **02.07.2010**

(51) Int Cl.:
***C07D 231/38*** (2006.01) ***C07C 275/30*** (2006.01)
***A61K 31/415*** (2006.01) ***A61P 35/00*** (2006.01)

(86) Numéro de dépôt international:
**PCT/FR2010/051394**

(87) Numéro de publication internationale:
**WO 2011/001122 (06.01.2011 Gazette 2011/01)**

(54) **Dérivés de pyrazoles, leur préparation et leur application en thérapeutique**

Pyrazolderivate, ihre Herstellung und ihre Verwendung als Arzneimittel

Pyrazole derivatives, their preparation and therapeutical use

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **03.07.2009 FR 0903270**

(43) Date de publication de la demande:
**09.05.2012 Bulletin 2012/19**

(73) Titulaire: **SANOFI**
**75008 Paris (FR)**

(72) Inventeurs:
- **ABECASSIS, Pierre-Yves**
**F-75013 Paris (FR)**
- **DESMAZEAU, Pascal**
**F-75013 Paris (FR)**
- **TABART, Michel**
**F-75013 Paris (FR)**

(74) Mandataire: **Gaslonde, Aude et al**
**Sanofi**
**Département Brevets**
**54, rue La Boétie**
**75008 Paris (FR)**

(56) Documents cités:
**WO-A-2008/065282**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 448 924 B1

## Description

La présente invention se rapporte à des dérivés de pyrazoles, à leur préparation et à leur application en thérapeutique.

Plus particulièrement, et selon un premier aspect, l'invention concerne de nouveaux pyrazoles substitués spécifiques présentant une activité anticancéreuse, via la modulation de l'activité de protéines, en particulier des kinases.

Les protéines kinases sont une famille d'enzymes qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis- à- vis de kinases. Parmi les kinases pour lesquelles une modulation de l'activité est recherchée, on peut citer KDR, Tie2, VEGFR- 1 (FLT1) , VEGFR- 3 (Flt4) , PDGFR, FGFR. Les kinases KDR et/ou Tie2 sont préférées.

Il est connu selon la demande de brevet publiée sous le WO08065282 des composés répondant à la formule générale (I) suivante :

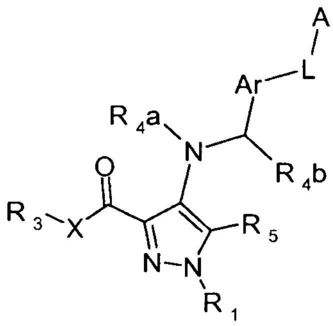

(I)

Formule (I)

dans laquelle:

1) A et Ar sont indépendamment sélectionnés dans le groupe constitué par : aryle, hétéroaryle, aryle substitué, hétéroaryle substitué;

2) L est sélectionné dans le groupe constitué par: NH-CO-NH et O-CO-NH;

3) $R_1$ est sélectionné dans le groupe constitué par : H, $R_6$, $COR_6$, $SO_2R_6$, dans lequel $R_6$ est choisi parmi H, $OR_7$, $NR_8R_9$, alkyle, cycloalkyle, hétérocyclyle, hétérocyclyle substitué, aryle, aryle substitué, hétéroaryle, hétéroaryle substitué, dans lequel $R_7$ est choisi parmi H, phényle, alkyle, et dans lequel $R_8$ et $R_9$ sont indépendamment sélectionnés dans le groupe constitué par H, alkyle, cycloalkyle, hétérocyclyle, hétérocyclyle substitué, aryle, aryle substitué, hétéroaryle, hétéroaryle substitué ou bien $R_8$ et $R_9$ sont liés entre eux pour former un cycle saturé de 5 à 8 chaînons contenant de 0 à 3 hétéroatomes choisis parmi O, S et N ;

4) X est sélectionné dans le groupe constitué par: O et NH ;

5) $R_3$ est sélectionné dans le groupe constitué par : H, alkyle, alkyle substitué, cycloalkyle , cycloalkyle substitué ;

6) R4a est sélectionné dans le groupe constitué par : H et (C1- C4) alkyle ;

7) R4b est sélectionné dans le groupe constitué par : H et (C1- C4) alkyle ;

8) $R_5$ est sélectionné dans le groupe constitué par : H, halogène, $R_{10}$, CN, O ($R_{10}$) , OC (O) ($R_{10}$) , OC (O) N ($R_{10}$) ($R_{11}$) , OS ($O_2$) ($R_{10}$) , N ($R_{10}$) ($R_{11}$) , N=C ($R_{10}$) ($R_{11}$) , N ($R_{10}$) C (O) ($R_{11}$) , N ($R_{10}$) C (O) O ($R_{11}$) , N ($R_{12}$) C (O)

N ($R_{10}$) ($R_{11}$) , N ($R_{12}$) C (S) N ($R_{10}$) ($R_{11}$) , N ($R_{10}$) S ($O_2$) ($R_{11}$) , C (O) ($R_{10}$) , C (O) O ($R_{10}$) , C (O) N ($R_{10}$) ($R_{11}$) , C (=N ($R_{11}$) ) ($R_{10}$) , C (=N ($OR_{11}$) ) ($R_{10}$) , S ($R_{10}$) , S (O) ($R_{10}$) , S ($O_2$) ($R_{10}$) , S ($O_2$) O ($R_{10}$) , S ($O_2$) N ($R_{10}$) ($R_{11}$) ; dans lequel chaque $R_{10}$, $R_{11}$, $R_{12}$ est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué.

Dans ce brevet de préférence X est O, R3 est méthyle, R4a et R4b sont H ; L est NHCONH ; A est phényle ; Ar est phényle ; mais dans cette demande aucun exemple ne décrit la substitution de Ar et son effet sur la pharmacocinétique.

La présente invention a pour objet deux composés inclus dans la demande précédente et répondant à la formule (I)

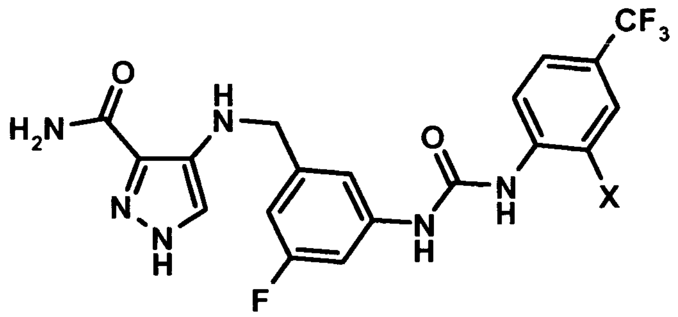

(I)

dans laquelle :

X représente Cl ou F

Les composés de formule (I) peuvent exister à l'état de bases ou de sels d'addition à des acides. De tels sels d'addition font partie de l'invention.

Les composés sous leurs deux formes tautomères indiquées ci-dessous appartiennent à l'invention :

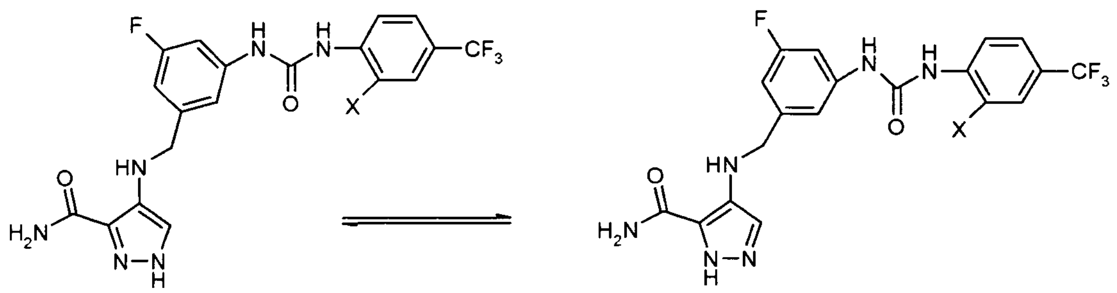

Ces sels peuvent être préparés avec des acides pharmaceutiquement acceptables, mais les sels d'autres acides utiles, par exemple, pour la purification ou l'isolement des composés de formule (I) font également partie de l'invention. Parmi les sels utilisables on peut notamment citer le chlorhydrate.

Les composés de formule (I) peuvent également exister sous forme d'hydrates ou de solvats, à savoir sous forme d'associations ou de combinaisons avec une ou plusieurs molécules d'eau ou avec un solvant.

Parmi les composés de formule (I) objets de l'invention, on peut notamment citer les composés suivants :

- 4- {3- [3- (2- chloro- 4- trifluorométhyl- phényl)- uréido]- 5- fluoro- benzylamino}- 1H- pyrazole- 3- carboxamide et son chlorhydrate
- 4- {3- [3- (2- fluoro- 4- trifluorométhyl- phényl)- uréido]- 5- fluoro- benzylamino}- 1H- pyrazole- 3- carboxamide et son chlorhydrate

Conformément à l'invention, on peut préparer les composés de formule générale (I) selon le procédé des revendications telles que déposées.

Dans les schémas qui suivent les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet les composés de formules

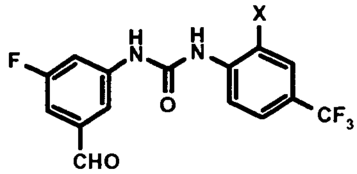

et

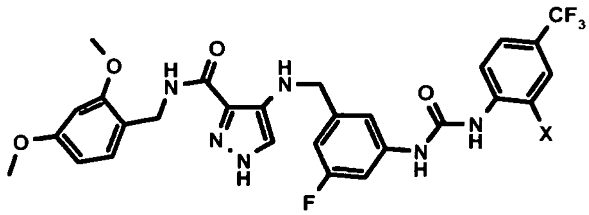

Dans lesquels X représente F ou Cl.

Ces composés sont utiles comme intermédiaires de synthèse des composés de formule (I) .

Les exemples suivants décrivent la préparation des composés conformes à l'invention. Ces exemples ne font qu'illustrer la présente invention.

**Procédé de synthèse des exemples**

Synthèse de la partie amino-pyrazole

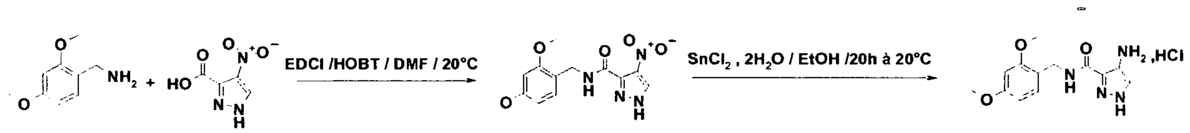


Synthèse de la partie diaryl-urée

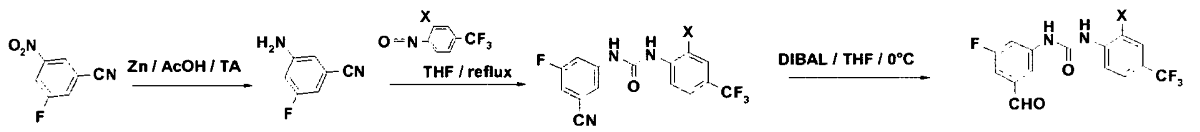


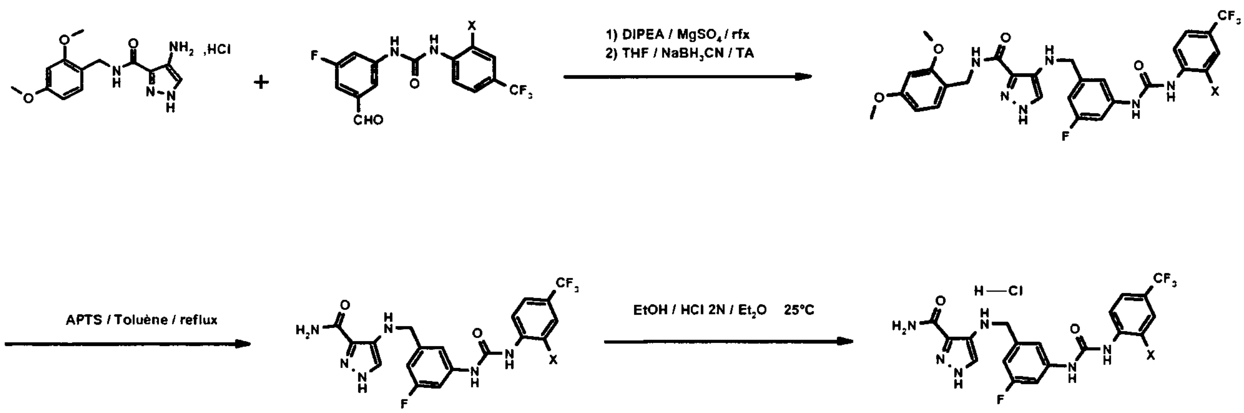


## Exemple1

**Chlorhydrate de 4-{3-[3-(2-chloro-4-trifluorométhyl-phényl)-uréido]-5-fluoro-benzylamino}-1H-pyrazole-3-carboxamide**

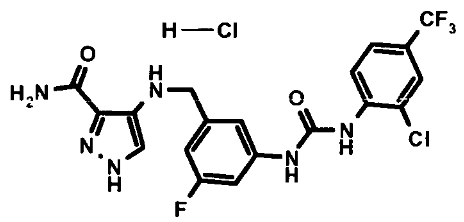

Une suspension de 1, 64 g (3, 48 mmole) de 4- {3- [3- (2- chloro- 4- trifluorométhyl- phényl)- uréido]- 5- fluoro-benzylamino}- 1 H- pyrazole- 3- carboxamide dans 50 mL d'éthanol est agitée à température ambiante sous atmosphère d'argon. Puis 35 mL (35 mmole) d'une solution d'acide chlorhydrique dans le diéthyle éther (1 N) sont ajoutés goutte à goutte. Le milieu réactionnel devient une solution limpide. Après 10 heures d'agitation à température ambiante les solvants sont évaporés à l'évaporateur rotatif sous pression réduite. Le résidu obtenu est agité dans 50 mL de diéthyle éther pendant 30 minutes.

Après filtration et séchage à l'étuve 1, 8 g de chlorhydrate de 4- {3- [3- (2- chloro- 4- trifluorométhyl- phényl)-uréido]- 5- fluoro- benzylamino}- 1H- pyrazole- 3- carboxamide sont obtenus sous forme de cristaux jaune pâle.
SM : Temps de rétention Tr (min) = 1, 01 ; [M+H] + m/z = 471 ; [M- H]- m/z = 469
1H NMR (400 MHz, DMSO- *d*) δ ppm 4.32 (s, 2 H) 6.89 (d large, *J*=9.6 Hz, 1 H) 7.16 (s large, 1 H) 7.18- 7.61 (m, 4 H) 7.68 (d large, *J*=8.9 Hz, 1 H) 7.86 (s large, 1 H) 8.44 (d, *J*=8.9 Hz, 1 H) 8.81 (m large, 1 H) 10.17 (m large, 1 H)
Point de fusion (Kofler) = 177°C

**4-{3-[3-(2-Chloro-4-trifluorométhyl-phényl)-uréido]-5-fluoro-benzylamino}-1H-pyrazole-3-carboxamide**

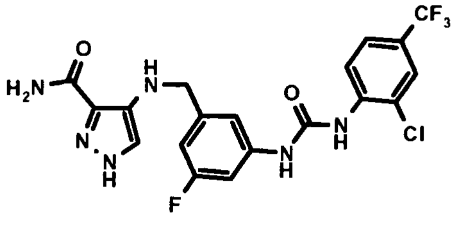

Une solution de 5, 9 g (9, 5 mmole) de 4- {3- [3- (2- chloro- 4- trifluorométhyl- phényl)- uréido]- 5- fluoro-benzylamino}- 1H- pyrazole- 3- (2, 4- diméthoxy- benzylamide) et 4, 5 g (24 mmole) d'acide *para*toluène sulfonique dans 400 mL de toluène est chauffée au reflux pendant 2 heures. Après décantation ! a solution toluènique est séparée d'une gomme jaune. La gomme est diluée dans 150 mL de méthanol et 500 mL d'acétate d'éthyle. Puis 500 mL d'eau sont ajoutés. La solution est alors refroidie puis alcalinisée avec 100 mL d'une solution aqueuse de potasse (10 N) . Après décantation la phase aqueuse est extraite avec une solution de 400 mL d'acétate d'éthyle et 100 mL de méthanol. Les phases organiques sont rassemblées et lavées avec 100 mL de solution saturée de chlorure de sodium, séchées sur sulfate de magnésium et concentrées sous pression réduite pour donner un solide jaune pâle qui est purifié sur 200 g de silice, élué avec une solution de dichlorométhane/méthanol/acétonitrile 80/10/10 (en volume) : 1, 77 g de 4- {3- [3- (2- chloro- 4- trifluorométhyl- phényl)- uréido]- 5- fluoro- benzylamino}- 1H- pyrazole- 3- carboxamide sont obtenus sous forme d'un solide blanc.
SM : Temps de rétention Tr (min) = 4, 29 [M+H] + m/z = 471 ; [M- H]- m/z = 469
1H NMR (400 MHz, DMSO- *d*) δ ppm 4.19 (d, *J*=6.7 Hz, 2 H) 5.74 (m large, 1 H) 6.80 (d large, *J*=9.6Hz, 1 H) 6.95- 7.11 (m, 3 H) 7.24 (m étalé, 1 H) 7.42 (dt, *J*=11.3, 2.3 Hz, 1 H) 7.67 (d large, *J*=8.9 Hz, 1H) 7.86 (s large, 1 H) 8.44 (d, *J*=8.9 Hz, 1 H) 8.70 (s large 1 H) 9.92 (s large, 1 H) 12.57 (s large, 1 H)
Point de fusion (Kofler) : 220°C

**4-{3-[3-(2-Chloro-4-trifluorométhyl-phényl)-uréido]-5-fluoro-benzylamino}-1H-pyrazole-3-(2,4-diméthoxy-benzylamide)**

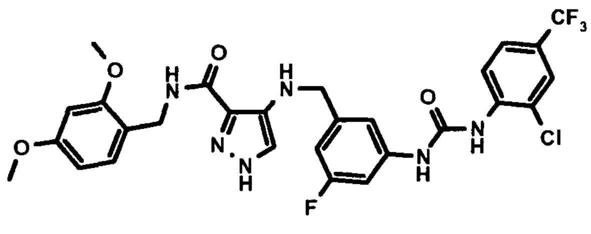

Une solution de 10 g (32 mmole) de chlorhydrate de 4- amino- 1H- pyrazole- 3- (2, 4- diméthoxy- benzylamide) et de 5, 9 mL (35, 2 mmole) de diisopropyléthylamine dans 300 mL de tétrahydrofurane est agitée à température ambiante sous atmosphère d'argon. 3, 85 g (35 mmole) de sulfate de magnésium et 12, 70 g (35, 2 mmole) de 1- (2- chloro- 4- trifluorométhyl- phényl)- 3- (3- fluoro- 5- formyl- phényl)- urée sont ajoutés. Le mélange réactionnel est alors chauffé au reflux pendant 14 heures. Le mélange est ensuite refroidi à 25°C puis 10, 08 g (160 mmole) de cyanoborohydrure de sodium sont additionnés lentement. Après 12 heures d'agitation à température ambiante, le mélange est concentré à sec à l'évaporateur rotatif. La gomme obtenue est agitée avec 300 mL d'eau et 500 mL de solution aqueuse de soude (1 N) . Cette suspension est agitée pendant 30 minutes avec 1 L de dichlorométhane. Après filtration sur verre fritté N°3, le solide obtenu est rincé avec 2 fois 500 mL d'eau puis séché à l'étuve sous vide à 40°C. Le solide est alors récristallisé dans 800 mL de méthanol, à chaud, pour donner 8, 3 g de 4- {3- [3- (2- chloro- 4- trifluorométhyl- phényl)-uréido]- 5- fluoro- benzylamino}- 1H- pyrazole- 3- (2, 4- diméthoxy- benzylamide) sous forme d'une poudre blanche.
SM : Temps de rétention Tr (min) = 4, 97 ; [M+H] +m/z = 621 ; [M- H]- m/z = 619
1 H NMR (400 MHz, DMSO- *d*) δ ppm 3.73 (s, 3 H) 3.81 (s, 3 H) 4.19 (d, J=6.7 Hz, 2 H) 4.33 (d, *J*=6.7Hz, 2 H) 5.71 (t large, *J*=6.7 Hz, 1 H) 6.46 (dd, *J*=8.3, 2.3 Hz, 1 H) 6.55 (d, *J*=2.3 Hz, 1 H) 6.80 (d large, *J*=9.6 Hz, 1 H) 7.04 (s large, 1 H) 7.08 (s, 1 H) 7.10 (d, *J*=8.9 Hz, 1 H) 7.43 (dt, *J*=11.4, 2.3 Hz, 1 H) 7.67 (d large, *J*=8.9 Hz, 1 H) 7.86 (s large, 1 H) 7.94 (t large, *J*=6.7 Hz, 1 H) 8.45 (d, *J*=8.9 Hz, 1 H) 8.62 (m
étalé, 1 H) 9.79 (m étalé, 1 H) 12.60 (m étalé, 1 H)
Point de fusion (Kofler) : 168°C

**1-(2-Chloro-4-trifluorométhyl-phényl)-3-(3-fluoro-5-formyl-phényl)-urée**

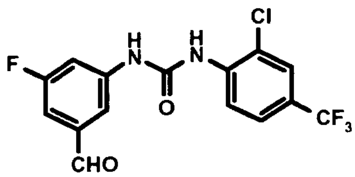

Une solution de 33, 15 g (92, 68 mmole) de 1- (2- chloro- 4- trifluorométhyl- phényl)- 3- (3- cyano- 5- fluoro-phényl)- urée dans 600 mL de tétrahydrofurane est agitée à- 10°C sous atmosphère d'argon. Puis 230 mL d'une solution d'hydrure de di- isobutyl aluminium à 20% dans le toluène sont ajouté avec un "goutte à goutte" régulier. Le mélange réactionnel est agité à température ambiante pendant 12 heures. Puis 80 mL supplémentaires d'hydrure de diisobutylaluminium sont ajoutés à- 10°C. Après 14 heures d'agitation à température ambiante le milieu réactionnel est concentré à sec à l'évaporateur rotatif pour donner une huile épaisse qui est additionnée lentement sous agitation avec 500 g de glace et 100 mL d'acide acétique à 100%. La suspension obtenue est filtrée. Le solide est alors dilué dans 2 fois 800 mL d'acétate d'éthyle et la solution organique est lavée avec 600 mL de solution saturée de chlorure de sodium, séchée sur sulfate de magnésium filtrée, concentrée à sec à l'évaporateur rotatif et séchée à l'étuve sous vide à 40°C pour donner 29, 57 g de 1- (2- chloro- 4- trifluorométhyl- phényl)- 3- (3- fluoro- 5- formyl- phényl)- urée sous forme d'une poudre jaune pâle.
SM : Temps de rétention Tr (min) = 4, 86 ; [M+H] + m/z = 361 ; [M- H]- m/z = 359
1 H NMR (400 MHz, DMSO- *d*) δ ppm 7.35 (ddd, *J*=8.4, 2.3, 1.8 Hz, 1 H) 7.67- 7.75 (m, 2 H) 7.78 (t, *J*=1.8 Hz, 1 H) 7.88 (d, *J*=1.8 Hz, 1 H) 8.45 (d, *J*=8.8 Hz, 1 H) 8.72 (s large, 1 H) 9.98 (d, *J*=1.8 Hz, 1 H) 10.07 (s large, 1 H)

**1-(2-Chloro-4-trifluorométhyl-phényl)-3-(3-cyano-5-fluoro-phényl)-urée**

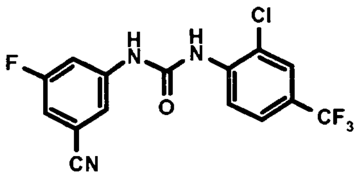

Une solution de 15 g (110, 2 mmole) de 5- fluoro- 3- cyanoaniline dans 150 mL de tétrahydrofurane est agitée à température ambiante sous atmosphère d'argon. Puis 17, 5 mL (121, 22 mmole) de 2- chloro- 4- trifluorométhylphénylisocyanate sont ajoutés. Le milieu réactionnel est chauffé au reflux pendant 3 heures puis concentré à sec à l'évaporateur rotatif. Le résidu solide obtenu est recristallisé à chaud dans 60 mL d'acétate d'éthyle pour donner 33, 25 g de 1- (2- chloro- 4- trifluorométhyl- phényl)- 3- (3- cyano- 5- fluoro- phényl)- urée sous forme d'un solide blanc.
SM : temps de rétention Tr (min) = 4, 97 ; $[M+H]^+$: m/z 356
Point de fusion (Kofler) : 286°C
La 5- fluoro- 3- cyanoaniline est commerciale.

**Chlorhydrate de 4-amino-1 H-pyrazole-3-(2,4-diméthoxy-benzylamide)**

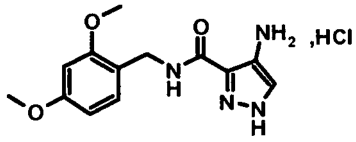

Une suspension de 6, 12 g (20 mmole) de 4- nitro- 1H- pyrazole- 3- (2, 4- diméthoxybenzylamide) dans 340 mL d'éthanol est agitée à température ambiante. Puis 15, 8 g (70 mmole) de di- hydrate de chlorure d'étain sont additionnés en 5 minutes. Le mélange réactionnel est agité 14 heures à température ambiante puis concentré à sec à l'évaporateur rotatif. Le résidu obtenu est agité avec 330 mL de solution aqueuse saturée en hydrogénocarbonate de sodium et 300 ml de dichlorométhane. Après décantation la phase organique est extraite avec 2 fois 150 mL de dichlorométhane. Les phases organiques sont réunies, lavées avec 150 mL de solution saturée en chlorure de sodium, séchées sur sulfate de magnésium. Après concentration à l'évaporateur rotatif, 4, 57 g de chlorhydrate de 4- amino- 1 H- pyrazole-3- (2, 4- diméthoxy- benzylamide) sont obtenus sous forme d'un solide de couleur violacée.
SM : IE: [M] +. m/z = 276 ; pic de base m/z = 151
1H NMR (400 MHz, DMSO- *d*) δ ppm 3.73 (s, 3 H) 3.81 (s, 3 H) 4.31 (d, J=6.2 Hz, 2 H) 4.56 (m étalé, 2 H) 6.46 (dd, *J*=8.3, 2.9 Hz, 1 H) 6.55 (d, *J*=2.9 Hz, 1 H) 7.06- 7.13 (m, 2 H) 7.84 (t, *J*=6.2 Hz, 1 H) 12.50 (m étalé, 1 H)
Point de fusion (Buchi) : 186°C

**4-Nitro-1H-pyrazole-3-(2,4-diméthoxybenzylamide)**

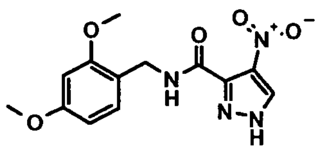

Une solution de 14, 65 g (76, 4 mmole) de 1- (3- diméthylaminopropyl)- 3- éthylcarbodiimide dihydrate, de 10, 32 g (76, 4 mmole) de 1- hydroxybenzotriazole dans 50 mL de diméthylformamide est agité à température ambiante. 11, 7 g (70, 03 mmole) de 2, 4- diméthoxybenzylamine sont ajoutés puis 10, 2 g d'acide 4- nitro- 3- pyrazole carboxylique par petites portions. Après 16 heures d'agitation à température ambiante, le milieu réactionnel est versé dans 500 mL d'eau. La suspension est filtrée puis lavée avec 2 fois 250 mL d'eau. Le solide obtenu est séché à l'étuve sous vide à 40°C pour donner 18, 58 g de 4- nitro- 1H- pyrazole- 3- (2, 4- diméthoxybenzylamide) sous forme d'un solide blanc.
1 H NMR (400 MHz, DMSO- *d*) δ ppm 3.75 (s, 3 H) 3.80 (s, 3 H) 4.35 (d, *J*=5.9 Hz, 2 H) 6.50 (dd, *J*=8.3, 2.4 Hz, 1 H) 6.56 (d, *J*=2.4 Hz, 1 H) 7.21 (d, *J*=8.3 Hz, 1 H) 8.71 (s large., 1 H) 8.88 (t large, *J*=5.9 Hz, 1 H) 14.13 (m étalé, 1 H)
SM (ES+/- ) Temps de rétention Tr (min) = 3, 23 ; [M+H] + m/z = 307 ; [M- H]- m/z = 305
Point de fusion (Kofler) : 192°C

L'acide 4- nitro- 3- pyrazole carboxylique est commercial.

**Exemple 2**

**Chlorhydrate de 4-{3-[3-(2-fluoro-4-trifluorométhyl-phényl)-uréido]-5-fluoro-benzylamino}-1H-pyrazole-3-carboxamide**

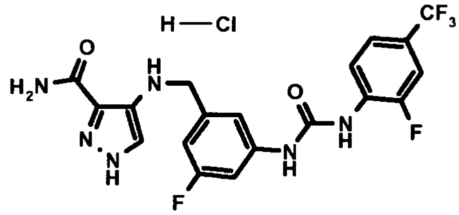

Une suspension de 1, 85 g (0, 40 mmole) de 4- {3- [3- (2- fluoro- 4- trifluorométhyl- phényl)- uréido]- 5- fluorobenzylamino}- 1 H- pyrazole- 3- carboxamide dans 40 mL d'éthanol est agitée à température ambiante sous atmosphère

d'argon. 20 mL (40 mmole) d'une solution d'acide chlorhydrique dans le diéthyl éther (1 N) sont ajoutés goutte à goutte. Le milieu réactionnel devient une solution limpide. Après 12 heures d'agitation à température ambiante les solvants sont évaporés à l'évaporateur rotatif sous pression réduite. Le résidu obtenu est agité dans 200 mL de diéthyl éther pendant 30 minutes.

Après filtration et séchage à l'étuve 1, 75 g de chlorhydrate de 4- {3- [3- (2- fluoro- 4- trifluorométhyl- phényl)- uréido]- 5- fluoro- benzylamino}- 1H- pyrazole- 3- carboxamide sont obtenus sous forme de cristaux jaune pâle.
$_1$H NMR (400 MHz, DMSO- $d_6$) δ ppm 4.24 (s, 2 H) 6.83 (d, *J*=9.0 Hz, 1 H) 7.08 (s 1H) 7.18 (br. s., 1H) 7.23 (s, 1 H) 7.35 (br. s., 2 H) 7.42 (dt, *J*=11.3, 2.0 Hz, 1 H) 7.54 (d, *J*=8.8 Hz, 1 H) 7.69 (dd, *J*=11.2, 1.5 Hz, 1 H) 8.41 (t, *J*=8.2 Hz, 1 H) 8.99 (s, 1 H) 9.54 (s, 1 H)
SM : Temps de rétention Tr (min) = 0, 97 ; [M+H] + m/z = 455
Point de fusion (Kofler) : 184°C (avec décomposition)

**4-{3-[3-(2-Fluoro-4-trifluorométhyl-phényl)-uréido]-5-fluoro-benzylamino}-1H-pyrazole-3-carboxamide**

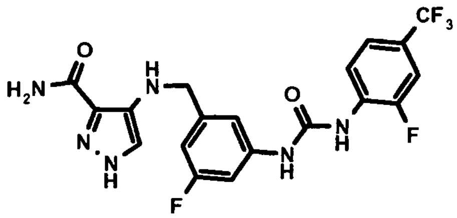

Une solution de 22, 3 g (36, 89 mmole) de 4- {3- [3- (2- fluoro- 4- trifluorométhyl- phényl)- uréido]- 5- fluoro- benzylamino}- 1H- pyrazole- 3- (2, 4- diméthoxy- benzylamide) et 17, 54 g (92, 22 mmole) d'acide *para*toluène sulfonique dans 600 mL de toluène est chauffée au reflux pendant 14 heures. Après décantation la solution toluènique est séparée d'une gomme jaune. La gomme est agitée pendant 2 heures dans 280 mL d'eau et 100 mL de soude (10 N) . La suspension est filtrée. Le solide obtenu est rincé avec 3 fois 350 mL d'eau puis séché pour donner un solide crème qui est purifié sur 350 g de silice, élué avec une solution de dichlorométhane/méthanol/acétonitrile 95/2, 5/2, 5 (en volume) : 3, 85 g de 4- {3- [3- (2- fluoro- 4- trifluorométhyl- phényl)- uréido]- 5- fluoro- benzylamino}- 1H- pyrazole- 3- carboxamide sont obtenus sous forme d'un solide blanc.
$_1$H NMR (400 MHz, DMSO- $d_6$) δ ppm 4.18 (d, J=5.9 Hz, 2 H) 5.68- 5.77 (m, 1 H) 6.80 (d, *J*=8.8 Hz, 1 H) 7.01- 7.08 (br. s., 1 H) 7.04 (s, 1 H) 7.06 (s, 1 H) 7.24 (br. s., 1H) 7.40 (dt, *J*=11.4, 2.0 Hz, 1 H) 7.54 (d, *J*=8.3 Hz, 1 H) 7.69 (d, *J*=11.5 Hz, 1 H) 8.41 (t, *J*=8.4 Hz, 1 H) 8.92 (br. s., 1 H) 9.40 (br. s., 1 H) 12.55 (br. s., 1 H)
SM : Temps de rétention Tr (min) = 4, 09 [M+H] + m/z = 455 ; [M- H]- m/z = 453
Point de fusion (Kofler) : 233°C

**4-{3-[3-(2-Fluoro-4-trifluorométhyl-phényl)-uréido]-5-fluoro-benzylamino}-1H-pyrazole-3-(2,4-diméthoxy-benzylamide)**

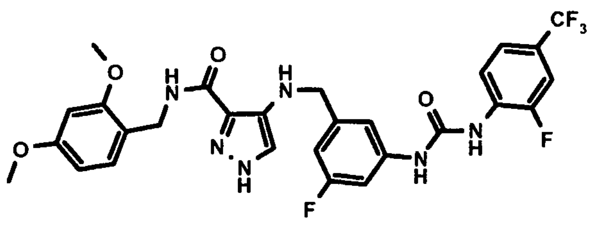

Une solution de 11, 40 g (36, 45 mmole) de chlorhydrate de 4- amino- 1H- pyrazole- 3- (2, 4- diméthoxy- benzylamide) et de 6, 65 mL (40, 09 mmole) de diisopropyléthylamine dans de 360 mL de tétrahydrofurane est agitée à température ambiante sous atmosphère d'argon. 4, 35 g (36, 45 mmole) de sulfate de magnésium et 13, 80 g (35, 2 mmole) de 1- (2- fluoro- 4- trifluorométhyl- phényl)- 3- (3- fluoro- 5- formyl- phényl)- urée sont ajoutés. Le mélange réactionnel est alors chauffé au reflux pendant 14 heures. Le mélange est ensuite refroidi à 25°C puis 11, 46 g (182, 25 mmole) de cyanoborohydrure de sodium sont additionnés lentement. Après 72 heures d'agitation à température ambiante, le mélange est concentré à sec à l'évaporateur rotatif La gomme obtenue est agitée avec 400 mL d'eau et

500 mL de solution de soude (1 N) . Cette suspension est agitée pendant 1 heure puis filtrée sur verre fritté N°3, le solide obtenu est rincé avec 3 fois 500 mL d'eau puis séché à l'étuve sous vide à 40°C pour donner 22, 45 g de 4- {3- [3- (2- fluoro- 4- trifluorométhyl- phényl)- uréido]- 5- fluoro- benzylamino}- 1H- pyrazole- 3- (2, 4- diméthoxy- benzylamide) sous forme d'un solide rosâtre.

$_1$H NMR (500 MHz, DMSO- $d_6$) δ ppm 3.73 (s, 3 H) 3.81 (s, 3 H) 4.18 (d, J=6.3 Hz, 2 H) 4.33 (s, 2 H)- 5.70 (t, *J*=6.2 Hz, 1 H) 6.47 (d, *J*=8.2 Hz, 1 H) 6.55 (s, 1 H) 6.79 (d, *J*=9.1 Hz, 1 H) 7.05 (s, 1 H) 7.07- 7.12 (m, 2 H) 7.41 (d, *J*=11.3 Hz, 1 H) 7.53 (d, *J*=8.2 Hz, 1 H) 7.68 (d, *J*=11.3 Hz, 1 H) 7.95 (br. s., 1 H)- 8.40 (t, *J*=8.2 Hz, 1 H) 9.25 (br. s., 2 H) 12.52 (br. s., 1 H)

SM : Temps de rétention Tr (min) = 4, 79 ; [M+H] + m/z = 605 ; [M- H]- m/z = 603

Point de fusion (Kofler) : 152°C

**1-(2-Fluoro-4-trifluorométhyl-phényl)-3-(3-fluoro-5-formyl-phényl)-urée**

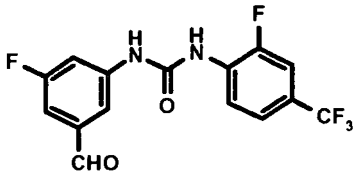

Une solution de 11, 90 g (34, 87 mmole) de 1- (2- fluoro- 4- trifluorométhyl- phényl)- 3- (3- cyano- 5- fluoro- phényl)- urée dans 150 mL de tétrahydrofurane est agitée à- 2°C sous atmosphère d'argon. Puis 86 mL d'une solution d'hydrure de di- isobutyl aluminium à 20% dans l'hexane sont ajouté avec un "goutte à goutte" régulier. Le mélange réactionnel est agité à température ambiante pendant 12 heures. Puis 60 mL supplémentaires d'hydrure de diisobutylaluminium sont ajoutés à- 2°C. Après 3 heures d'agitation à température ambiante le milieu réactionnel est concentré à sec à l'évaporateur rotatif pour donner une huile épaisse qui est additionnée lentement sous agitation avec 500 g de glace et 300 mL d'acide acétique à 100%. La suspension obtenue est filtrée. Le solide obtenu est lavé avec 4 fois 150 mL d'eau, essoré et séché à l'étuve sous vide à 40°C pour donner 13, 95 g de 1- (2- fluoro- 4- trifluorométhyl- phényl)- 3- (3- fluoro- 5- formyl- phényl)- urée sous forme d'un solide jaune pâle.

$_1$H NMR (400 MHz, DMSO- $d_6$) δ ppm 7.33- 7.38 (m, 1 H) 7.57 (d, J=8.8 Hz, 1 H) 7.69- 7.75 (m, 2 H)- 7.79 (s, 1 H) 8.41 (t, *J*=8.3 Hz, 1 H) 9.05 (br. s., 1 H) 9.65 (s, 1 H) 9.98 (d, *J*=1.7 Hz, 1 H)

SM : Temps de rétention Tr (min) = 4, 62 ; [M+H] + m/z = 345 ; [M- H]- m/z = 343

Point de fusion (Kofler) : 247°C

**1-(2-Fluoro-4-trifluorométhyl-phényl)-3-(3-cyano-5-fluoro-phényl)-urée**

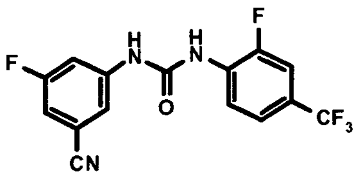

Une solution de 6, 15 g (45, 18 mmole) de 5- fluoro- 3- cyanoaniline dans 90 mL de tétrahydrofurane est agitée à température ambiante sous atmosphère d'argon. 4, 3 mL (36, 14 mmole) de disphosgène sont ajoutés goutte à goutte puis 30 mL (135, 54 mmole) de triéthylamine. Après 3 heures de reflux du mélange réactionnel, une solution de 7, 10 g (39, 64 mmole) de 4- amino- 3- fluoro- trifluorométhylbenzène dans 10 mL de tétrahydrofurane est ajoutée lentement. Le reflux est maintenu 2 heures de plus. Le milieu est alors agité dans 100 mL d'eau puis extrait avec 100 mL d'acétate d'éthyle. La phase organique est lavée avec 100 mL d'une solution saturée en chlorure de sodium, séchée sur sulfate de magnésium et concentré à sec à l'évaporateur rotatif. Le résidu solide obtenu est recristallisé à chaud dans 90 mL d'acétonitrile pour donner 10, 35 g de 1- (2- fluoro- 4- trifluorométhyl- phényl)- 3- (3- cyano- 5- fluoro- phényl)- urée sous forme d'un solide crème.

$_1$H NMR (400 MHz, DMSO- $d_6$) δ ppm 7.47 (d, *J*=8.3 Hz, 1 H) 7.57 (d, *J*=8.6 Hz, 1 H) 7.69 (s, 1 H) 7.70- 7.75 (m, 2 H) 8.37 (t, *J*=8.4 Hz, 1 H) 9.17 (br. s., 1 H) 9.63 (br. s., 1 H)

SM : temps de rétention Tr (min) = 1.1 ; $[M+H]^+$ : m/z 341.

Point de fusion (Kofler) : 253°C

Les produits de l'invention sont utiles comme agents inhibiteurs d'une ou plusieurs réactions catalysées par une kinase. KDR et/ou Tie2 sont des kinases pour lesquelles les produits de l'invention seront particulièrement utiles en tant qu'inhibiteurs.

Les raisons pour lesquelles ces kinases sont choisies sont données ci-après:

**KDR**

KDR (Kinase insert Domain Receptor) aussi appelée VEGF-R2 (Vascular Endothelial Growth Factor Receptor 2), est principalement exprimé dans les cellules endothéliales. Ce récepteur lie le facteur pro-angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. En plus de ce rôle central dans le processus dynamique angiogénique, des résultats récents suggèrent que l'expression de VEGF contribue à la survie des cellules tumorales après des chimio- et radiothérapies, soulignant la synergie potentielle d'inhibiteurs de KDR avec d'autres agents (Lee et al. Cancer Research, 2000, vol. 60, p.5565-5570).

**Tie2**

Tie- 2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïétine 1 ou Ang1) qui stimule l'autophosphorylation du récepteur et la signalisation cellulaire [S. Davis et al (1996) Cell 87, 1161- 1169] et l'antagoniste (angiopoïétine 2 ou Ang2) [P.C. Maisonpierre et al. (1997) Science 277, 55- 60] *.* L'angiopoïétine 1 peut avoir un effet synergique avec le VEGF dans les derniers stades de la néo- angiogénèse [AsaharaT. Circ. Res. (1998) 233- 240] . Les expériences de knock- out et les manipulations transgéniques de l'expression de Tie2 ou de Ang1 conduisent à des animaux qui présentent des défauts de vascularisation [D.J. Dumont et al (1994) Genes Dev. 8, 1897- 1909 *et* C. Suri (1996) Cell 87, 1171- 1180*]* . La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55- 60*]* *.* Lin et al (1997) J. Clin. Invest. 100, 8: 2072- 2078 *et* Lin P. (1998) PNAS 95, 8829- 8834*,* ont montré une inhibition de la croissance et de la vascularisation tumorale, ainsi qu'une diminution des métastases de poumon, lors d'infections adénovirales ou d'injections du domaine extracellulaire de Tie- 2 (Tek) dans des modèles de xénogreffes de tumeur du sein et de mélanome.

Pour les raisons qui suivent, les inhibiteurs de Tie2 peuvent être utilisés dans les situations où une néovascularisation ou angiogenèse se fait de façon inappropriée c'est-à-dire dans les cancers en général mais aussi dans des cancers particuliers tels que le sarcome de Kaposi ou l'hémoangiome infantile, l'arthrite rhumatoïde, l'osthéoarthrite et/ou ses douleurs associées, les maladies inflammatoires de l'intestin telle que la recto-colite hémorragique ou la maladie de Crohn's, les pathologies de l'oeil telle que la dégénérescence maculaire liée à l'âge, les rétinopathies diabétiques, l'inflammation chronique, le psoriasis.

L'angiogenèse est un processus de génération de nouveaux vaisseaux capillaires à partir des vaisseaux préexistants. L'angiogenèse tumorale (formation de néovaisseaux sanguins) , indispensable à la croissance tumorale, est également un des facteurs essentiels de la dissémination métastatique (Oncogene. 2003 May 19; 22 (20) : 3172- 9 ; Nat Med. 1995 Jan; 1 (1) : 27- 31.) .

Cette néo-vascularisation est due à la migration, puis à la prolifération et à la différenciation des cellules endothéliales sous l'influence de facteurs angiogéniques sécrétés par les cellules cancéreuses et les cellules du stroma (Recent Prog Horm Res. 2000;55:15-35; 35-6).

Le système angiopoïétine 1 / récepteur Tie2 joue un rôle prépondérant dans la maturation des vaisseaux en permettant le recrutement de cellules péri- endothéliales pour stabiliser le vaisseau (Cell. 1996 Dec 27; 87 (7) : 1161- 9, Recent Prog Horm Res. 2004; 59: 51- 71) . Ainsi il a été montré que l'administration de la forme recombinante soluble du domaine extracellulaire du récepteur Tie- 2 (exTek) inhibe l'angiogenèse tumorale dans des modèles de tumeurs murines ainsi que le développement métastatique (Pengnian Lin, Jake A. Buxton, Ann Acheson, Czeslaw Radziejewski, Peter C. Maisonpierre, George D. Yancopoulos, Keith M. Channon, Laura P. Hale, Mark W. Dewhirst, Samuel E. George, and Kevin G. Peters, Proc Natl Acad Sci USA. 1998 Jul 21; 95 (15) : 8829- 34 ; Cecilia Melani, Antonella Stoppacciaro, Chiara Foroni, Federica Felicetti, Alessandra Caré et MarioP. Colombo, Cancer immunol Immunother. 2004 Jul; 53 (7) : 600- 8) . Dans des cellules endothéliales en culture, la stimulation de Tie- 2 active la voie de la Pl3 kinase, de p42/p44

voies impliquées dans la prolifération et la migration cellulaire; de la synthèse de PAF (Cell Signal. 2006 Apr 14; ahead of print) voie impliquée dans l'activité pro- inflammatoire. La stimulation de Tie2 stimule la voie de l'Akt et inhibe l'apoptose (Laura M DeBusk, Dennis E Hallahan, Pengnian Charles Lin, Exp Cell Res. 2004 Aug 1; 298 (1) : 167- 77) une voie de transduction connue pour son importance dans la survie cellulaire.

L'ajout de Extek (récepteur soluble de Tie2) inhibe la formation de pseudo tubules des cellules endothéliales sur Matrigel (Cecilia Melani, Antonella Stoppacciaro, Chiara Foroni, Federica Felicetti, Alessandra Caré et MarioP. Colombo, Cancer immunol Immunother. 2004 Jul; 53(7): 600-8. Ces études indiquent que le système Tie-2/Angiopoiétine est nécessaire lors des premiers stades de la formation de bourgeons vasculaires dans les tissus adultes et qu'une fonction du récepteur Tie-2 est d'augmenter la survie des cellules endothéliales au cours de la formation des vaisseaux sanguins. De plus, l'angiopoietine-1 stimule la prolifération des cellules endothéliales lymphatiques ainsi que la lymphangiogenèse (développement des néovaisseaux lymphatiques) voie d'accès privilégiés pour le développement métastatique (Tohru....Morisada, Yuichi Oike, Yoshihiro Yamada, Takashi Urano, Masaki Akao, Yoshiaki Kubota, Hiromitsu Maekawa, Yoshishige Kimura, Masako Ohmura, Takeshi Miyamoto, Shiro Nozawa, Gou Young Koh, Kari Alitalo, and Toshio Suda, Blood. 2005 Jun 15; 105(12): 4649-56).

Parmi les enzypes ayant un Rôle dans l'angiogénèse on peut aussi citer PDGFRβ (Guzman and Laurence H. Hurley, Univ of Arizona Molecular Cloning of the Human PDGFR- beta Promoter and Targeting the G- Quadruplex-Forming Region to Control gene Expression ; Biomedical Drug Discovery, Genomics/ Gentics, Therapeutic) , FGFR1 (Somaia Elbauomy Elsheikh, Andrew R Green,[1] Maryou BK Lambros, Nicholas C Turner, Matthew J Grainge,[3] Des Powe,[1] Ian O Ellis, and Jorge S Reis- Filho, *FGFR1* amplification in breast carcinomas: a chromogenic *in situ* hybridisation analysis ; FLT1 (Shibuya M (2007) . "Vascular endothelial growth factor receptor- 1 (VEGFR- 1/Flt- 1) : a dual regulator for angiogenesis.". Angiogenesis 9 (4) : 225- 30; discussion 231 et VEGFR3 (Tamela, T. et al Blocking VEGFR- 3 suppresses angiogenic sprouting and vascular network formation, Nature 454, 656- 660 (20058) .

Les processus d'angiogenèse jouent ainsi un rôle prépondérant dans la progression de nombreuses tumeurs solides. De plus, il a été montré que la probabilité d'apparition de métastases augmente très fortement avec l'augmentation de la vascularisation de la tumeur primaire (Br J Cancer. 2002 May 20 ; 86(10): 1566-77. Le rôle potentiel d'agents pro-angiogéniques dans les leucémies et lymphomes a été également et plus récemment documenté. En effet de manière générale il a été rapporté que des clones cellulaires dans ces pathologies peuvent être soit détruits naturellement par le système immunitaire soit basculer dans un phénotype angiogénique qui favorise leur survie puis leur prolifération. Ce changement de phénotype est induit par une sur expression de facteurs angiogéniques notamment par les macrophages et/ou une mobilisation de ces facteurs à partir de la matrice extracellulaire (Thomas DA, Giles FJ, Cortes J, Albitar M, Kantarjian HM., Acta Haematol, (2001), vol 207, pp106-190.

Il existe une corrélation entre le processus d'angiogenèse de la moelle osseuse et les "extramedullar disease" dans les CML (chronic myelomonocytic leukemia). Différentes études démontrent que l'inhibition de l'angiogenèse, pourrait représenter une thérapeutique de choix dans cette pathologie (Leuk Res. 2006 Jan; 30(1): 54-9 ; Histol Histopathol. 2004 oct. ;19(4): 1245-60. De plus, il est fortement suggéré que l'activation du système Tie2/angiopoietine soit impliquée dans le développement de l'angiogenèse de la moelle osseuse chez les patients atteints de myélome multiple (Blood. 2003 Jul 15; 102(2): 638-45.

Détermination de l'activité des composés - Protocoles expérimentaux

## 1. KDR

L'effet inhibiteur des composés est déterminé dans un test de phosphorylation de substrat *in vitro* par une technique de scintillation (plaque 96 puits de type basic Flash Plate).

Le domaine cytoplasmique (résidus 790 à 1356) de l'enzyme KDR humaine a été cloné sous forme de fusion GST dans le vecteur d'expression baculovirus pFastBac. La protéine a été exprimée dans les cellules SF21, purifiée et activée par autophosphorylation. Le substrat est composé des résidus 658 à 850 de la PLCγ exprimée et purifiée sous forme de protéine de fusion GST.

L'activité kinase de KDR est mesurée dans du tampon 20 mM MOPS, 10 mM MgCl2, 10 mM MnCl2, 1 mM DTT, pH = 7.4. Les composés sont initialement dilués dans du DMSO 100% puis préparés en solution 10X dans du DMSO 30% - tampon 70%. 10 μl de solution 10X sont déposés puis 70 μl de tampon contenant 150 ng (1,6 pmole) d'enzyme KDR à 4°C. La réaction est démarrée en ajoutant 20 μl de solution contenant 2 μg (41 pmole) de substrat PLCγ, 0,5 μCi de $\gamma^{33}$P[ATP] et 2 μM d'ATP froid. La plaque est agitée. Après 30 minutes d'incubation à 37°C, le tampon d'incubation est retiré et les puits sont lavés trois fois avec 300 μl de PBS. La radioactivité est mesurée dans chaque puits en utilisant un compteur de radioactivité Trilux-βWallac.

Le bruit de fond est déterminé par la mesure de la radioactivité dans quatre puits différents contenant l'ATP (radiomarqué et froid) et le substrat, en absence d'enzyme et de composé. L'activité contrôle est mesurée dans quatre puits différents contenant tous les réactifs mais en l'absence de composé.

L'inhibition de l'activité KDR avec le composé de l'invention est exprimée en pourcentage d'inhibition de l'activité contrôle déterminée en l'absence de composé.

**2. Tie2**

L'effet inhibiteur des composés est déterminé dans un test de phosphorylation de substrat *in vitro* par une technique de scintillation (plaque 96 puits de type basic Flash Plate).

La séquence codante de Tie2 humain correspondant aux acides aminés du domaine intracellulaire 774-1124 a été introduite dans un vecteur d'expression baculovirus pFastBacGT sous forme de protéine de fusion GST. GST-Tie2 a été purifiée et activée par autophosphorylation. Le substrat est composé des résidus 658 à 850 de la PLCγ exprimée et purifiée sous forme de protéine de fusion GST.

L'activité kinase de Tie2 est mesurée dans un tampon MOPS 20mM pH 7.4, contenant 10 mM $MgCl_2$, 10 mM $MnCl_2$, 1 mM DTT. Les composés sont initialement dilués dans du DMSO 100% puis préparés en solution 10X dans du DMSO 30%, tampon 70%. 10 μl de solution 10X sont déposés puis 70 μl de tampon contenant 100 ng (1,5 pmole) d'enzyme Tie2 à 4°C. La réaction est démarrée en ajoutant 20 μl de solution contenant 2 μg (41 pmole) de substrat PLCγ, 0,5 μCi de $\gamma^{33}$[ATP] et 2 μM d'ATP froid. La plaque est agitée. Après 30 minutes d'incubation à 37°C, le tampon d'incubation est retiré et les puits sont lavés trois fois avec 300 μl de PBS. La radioactivité est mesurée dans chaque puits en utilisant un compteur de radioactivité Trilux-βWallac.

Le bruit de fond est déterminé par la mesure de la radioactivité dans quatre puits différents contenant l'ATP (radiomarqué et froid) et le substrat, en absence d'enzyme et de composé. L'activité contrôle est mesurée dans quatre puits différents contenant tous les réactifs mais en l'absence de composé.

L'inhibition de l'activité Tie2 est calculée et exprimée en pourcentage d'inhibition par rapport à l'activité contrôle déterminée en l'absence de composé.

**3.Screening de molécules en mesurant par marquage radioactif l'activité de phosphorylation de kinase FLTI an présence de substat PFCγ.**

L'activité kinase de FLT1 est mesurée de la même manière que l'inhibition de Tie2 avec un mélange réactionnel composé de 70 μL de tampon kinase contenant 13 nM d'enzyme FLT1 par puits

L'inhibition de l'activité FLT1 est calculée et exprimée en pourcentage d'inhibition par rapport à l'activité contrôle déterminée en l'absence de composé.

Le pourcentage d'inhibition correspondant à chaque concentration de molécules est calculé ainsi :

% inhibition = (moyenne des CPM des puits traités – moyenne des CPM des puits contrôles) / (CPM des puits non traités- moyenne des CPM des contrôles) x 100

**4. Screening de molécules mesurant par marquage radioactif l'activité de phosphorylation de kinase PDGFR en présence du substrat PLCγ**

Le test est réalisé de façon parallèle à celui réalisé avec l'enzyme FLT1 en utilisant à la place de 13 nM de Flt1; 16 nM d'enzyme PDGFR et dans les plaques 96 puits on mélange 18 μl d'Enzyme GST-PDGFR à 4 mg/ml (pour une pureté de 80%) lot VLT802

5. **Screening de molécules mesurant par marquage radioactif l'activité de phosphorylation de kinase FGFR en présence du substrat PLCγ**

Le test est réalisé de façon parallèle à celui réalisé avec l'enzyme FLT1 en utilisant à la place de 13 nM de Flt1; 27 nM d'enzyme FGFR et dans les plaques 96 puits on mélange 18μl d'Enzyme GST- FGFR à 1,1mg/ml (pour une pureté de 100%) : (Lot JCE3666)

**Résultats :**

Les composés des exemples de l'invention présente une concentration inhibant 50 % de l'activité de la kinase qui est généralement comprise entre 0.1 nM et 2 μM sur KDR et/ou TIE2, de préférence comprise entre 0.1 nM et 500 nM, et plus préférentiellement comprise entre 0.1 nM et 50 nM. Les valeurs du tableau 1, ci-dessous, sont données à

titre d'illustration.

| COMPOSES | KDR nM | Tie2 nM | PDGFRβ nM | FGFR1 nM | FLT1 nM | VEGFR3 nM |
|---|---|---|---|---|---|---|
| 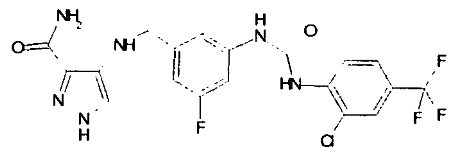 | 3 | 36 | 11 | 54 | 3 | 3 |
| 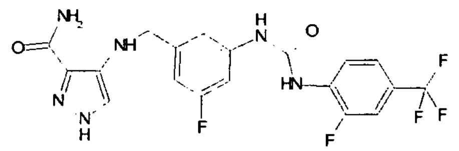 | 2 | 39 | 20 | 92 | 1.3 | |

Les composés selon l'invention ont fait l'objet d'essais pharmacologiques permettant de déterminer leur clairance hépatique.
Évaluation de la clairance intrinsèque des composés en utilisant des hépatocytes humains : protocole expérimental.
NB : Le test *in vitro* décrit ci-dessous sur des cellules de foie humain est utilisé pour prédire un paramètre pharmacocinétique important: la métabolisation par le foie pour un composé donné quand il est administré à l'homme.

Condition de culture :

Les incubations des préparations cryopréservées d' hépatocytes humains ( d'IVT: IVT-TLN-180608 provenant de In Vitro Technologies, inc, Baltimore, Maryland, USA et des préparations fraîches d' hépatocytes humains (de Biopredic : HEP200239) ont été faites dans des plaques comportant 48 puits enduits de collagène.

Conditions expérimentales :

Les cinétiques ont été démarrée par l'addition, dans le milieu de culture, des composés à une concentration finale de 5μM, en l'absence ou en la présence de Kétoconazole 10μM (inhibiteur de CYP3A4Le volume d'incubation est de 100 μL, la durée d'incubation : 0 - 24 heures (points cinétiques habituels : 0-0.5 - 1 - 2 - 4 - 6 - 8 - 24 heures).
Les cinétiques ont été arrêtées par l'addition de d'acétonitrile/eau, avec la corticostérone, comme standard interne. Les cellules ont été détachées puis lysées. Les milieux intracellulaires et extracellulaires ont été réunis et congelés (- 20°C) afin d'être stockés jusqu'à l'analyse LC-MS/MS.

Méthode analytique de LC-MS/MS:

Les milieux intracellulaires et extracellulaires réunis ont été décongelés, soumis aux ultrasons, agités par vortex et centrifugés à 3000g pendant 20 minutes. Les surnageants ont été injectés et analysés par LC-MS/MS.

Traitement des données et « classement »

- La vitesse in vitro initiale maximale a été calculée pour les métabolites spécifiques des cytochromes P450 et exprimée en nmole/heure/million de cellules.

$$V = [\text{concentration à } T(n) - \text{concentration à } T(n-1)] / [T(n) - T(n-1)]$$

La clairance intrinsèque a été déterminée et exprimée en mL/heure/million de cellule

$$Cl_{int} = \text{dose}/AUC0\text{-}24h$$

Dose = quantité à T (0) (nmoles/million de cellules)

AUC0-24h: calculé par WinNonlin, avec une analyse non compartimentée, modélisant une injection intraveineuse de type bolus

Le classement de la clairance intrinsèque a été défini comme suit:

$Cl_{int}$ < 0.040 mL.$hr^{-1}$.$10^{-6}$ cellule : clairance intrinsèque intermédiaire
0.040< $Cl_{int}$< 0.120 mL.$hr^{-1}$.$10^{-6}$ cellule: clairance intrinsèque faible
$Cl_{int}$ >0.040 mL.$hr^{-1}$.$10^{-6}$ cellule : clairance intrinsèque élevée

Information démographique sur le donneur:

Préparations d' hépatocytes humains cryopréservés :

D'IVT: • IVT, groupe TLN : Homme, Caucasien, 25 ans

Préparations d' hépatocyte humain frais:

De Biopredic : • HEP200239 : Femme, inconnu, 58 ans

Sur ce test, la valeur de clairance intrinsèque du composé décrit à l'exemple1 est de 0.057 mL/heure/million de cellule (valeur classée intermédiaire avec une faible variabilité inter-individuelle).
La valeur de clairance intrinsèque du composé décrit à l'exemple2 est de 0.055 mL/heure/million de cellule (valeur classée intermédiaire avec une faible variabilité inter-individuelle).

D'autres essais consistant à mesurer l'activité *in vivo* des composés de l'invention sur des tumeurs du colon ont été effectués.

Cette activité sur les tumeurs du colon des composés de l'invention a été étudiée sur le mélanome B16. En comparaison le produit de l'exemple 19 de la demande WO08065282 a été testé.
L'efficacité d'un produit peut être déterminée in vivo par différents critère on peut la déterminer par le pourcentage d'inhibition tumorale %T/C qui représente le rapport entre le poids moyen des tumeurs du groupe traité (T) et le poids moyen des tumeurs du groupe témoin (C) au jour 12 ou 13 du traitement. On considère un produit actif quand le rapport T/C est inférieur à 42% et un produit ayant une haute activité antitumorale quand le T/C est inférieur à 10%. (Corbett TH and coll., Cancer Research, 42, 1707-1715 (1982).

Pour mettre en évidence l'efficacité d'un composé, on peut aussi déterminer le long10 cellules tuées qui est déterminé selon la formule suivante :

$$\log_{10} \text{ des cellules tuées} = \text{T-C (jours)}/3.32 \times T_d$$

dans laquelle T - C représente le délai de croissance des cellules qui est le temps moyen, en jours, pour que les tumeurs du groupe traité (T) et les tumeurs du groupe témoin (C) aient atteint une valeur prédéterminée (750 mg par exemple) et $T_d$ représente le temps, en jours, nécessaire au doublement du volume de la tumeur chez les animaux témoins [T.H. CORBETT et coll., Cancer, 40, 2660-2680 (1977) ; F.M. SCHABEL et coll., Cancer Drug Development, Part B, Methods in Cancer Research, 17, 3-51, New-York, Academic Press Inc. (1979)] Un produit est considéré comme actif si $\log_{10}$ des cellules tuées est supérieur ou égal à 0,7. Un produit est considéré comme très actif si le $\log_{10}$ des cellules tuées est supérieur à 2,8.

L'efficacité des composés sur les tumeurs solides peut être déterminée expérimentalement de la manière suivante:

Les animaux soumis à l'expérience, généralement des souris femelles C57BL/6, sont greffés bilatéralement par voie sous-cutanée avec 30 à 60 mg d'un fragment de tumeur humaine B 16 (Référence de la tumeur.) au jour 0. Les animaux portant les tumeurs sont randomisés avant d'être soumis aux divers traitements et contrôles. Dans le cas de traitement de tumeurs de la présente invention, on a initié le traitement à un stade précoce, 3 à 4 jours après l'implantation. Les animaux qui ont subi le traitement avec les composés présentaient un poids d'environ 20 g,. Des animaux porteurs de tumeurs ont également été soumis aux mêmes traitements avec l'excipient seul afin de pouvoir dissocier l'effet toxique de l'excipient de l'effet propre de la chimiothérapie sur la tumeur. Les administrations des composés ont été faites par voie orale aux doses indiquées dans les tableaux et avec les excipients indiqués dans les tableaux selon une double administration quotidienne. Ces administrations ont été réalisées pendant 8 à 11 jours selon les études, après l'implantation de la tumeur.

Les tumeurs sont mesurées deux ou trois fois par semaine jusqu'à ce que la tumeur atteigne environ 2 g ou

jusqu'à la mort de l'animal si celle-ci survient avant que la tumeur atteigne 2 g. Les animaux sont autopsiés lors du sacrifice.

L'activité antitumorale est déterminée en fonction des différents paramètres enregistrés.

A titre d'exemples, sont donnés, dans les tableaux suivants les résultats obtenus avec les composés de l'invention utilisés à leur dose optimale.

Ainsi l'invention a pour objet des médicaments qui comprennent un composé de formule (I), ou un sel d'addition de ce dernier à un acide pharmaceutiquement acceptable.

Selon un autre de ses aspects, l'invention concerne les composés répondant à la formule (I) pour leur application comme médicament dans le traitement du cancer. Les composés selon l'invention peuvent être utilisés pour la préparation de médicaments, en particulier de médicaments inhibiteurs de l'angiogénèse.

Ces médicaments trouvent leur emploi en thérapeutique, notamment dans le traitement de tumeurs cancéreuses, notamment de tumeurs solides.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, les formes d'administration transdermique, sous-cutanée, intramusculaire ou intraveineuse.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Par voie orale, la dose de principe actif administrée par jour peut atteindre 1200 mg/kg, en une ou plusieurs prises.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

**EVALUATION DE L'EXEMPLE 1 CONTRE LE MELANOME MURIN PRECOCE B16**

| Composé p.o. (jour d'administration) | Dosage en mg/kg/ adm | Dose totale en mg/kq | Mort due au produit (jour de la mort) | %BWC (jour du nadir) | Poids moyen de la tumeur en mg au jour 13 (range) | T/C en % jour 13 | T-C en jours 750 mg | Log de cellule tuée totale | Commentaires |
|---|---|---|---|---|---|---|---|---|---|
| **Exemple 1** | **160.0** | **3178[b]** | **0/5** | **-2.3 (6)** | **23 (0-89)** | **2** | **6.8** | **2.6** | **HDT active** |
| **Jours 4-14 (2x/j)[a]** | **99** | **1966[b]** | **0/5** | **-1.0(5)** | **72 (0-75)** | **5** | **6.2** | **2.3** | **Active** |
| | **62** | **1232[b]** | **0/5** | **+1.8 (5)** | **128 (75-273)** | **10** | **6.2** | **2.3** | **Active** |
| **Jour 4-14** | **44** | **481[b]** | **0/5** | **+10.6(15)** | **686 (243-1418)** | **51** | **-** | **-** | **Inactive** |
| **Excipient PO 4-14 (2x/j)** | | | **0/10** | **+11.9(15)** | **1334 (496-1650[c])** | | | | |

BCM-1948. Temps de doublement de la tumeur = 0.8 jour. Formulation: Exemple 1 = 98% PEG200, 2% PS80. Abréviations utilisées : BWC= body weight change, HNTD= highest nontoxic dose, HDT = highest dose tested. [a] Une fois par jour pour jours 9 et 10. [b] La dose a été réduite par accident de 7% le jour 7. Calculé sur 9 souris du à une mort accidentelle le jour 8.
p.o. = *per os*

**EVALUATION DE L'EXEMPLE 2 CONTRE LE MELANOME MURIN PRECOCE B16**

| Composé p.o. (jour d'administration) | Dosage en mg/kg/adm | Dose totale en mg/kg | Mort due au produit (jour de la mort) | %BWC (jour du nadir) | Poids moyen de la tumeur en mg au jour 11 (range) | T/C en % jour 11 | T-C en jours 750 mg | Log de cellule tuée totale | Commentaires |
|---|---|---|---|---|---|---|---|---|---|
| Exemple 2 | 160.0 | 2560.0 | 6/6(10, 2j11, 13, 2j14) | -26,5(13) | - | - | - | - | Toxique |
| Jour 3-10 (2x/j) | 99.2 | 1587,2 | 0/6 | -8,1(11) | 0(0-14) | 0 | 5,4 | 1,8 | HNTD active |
| | 61.5 | 984 | 0/6 | +3,9 (11) | 46 (0-95) | 6 | 5.0 | 1,7 | Active |
| Jour 3-10 | 44.0 | 352.0 | 0/6 | +6,6(11) | 201 (23-822) | 27 | 3.0 | 1.0 | Active |
| Excipient PO 3-10 (2x/j) | | | 0/10 | +9,3(11) | 736 (222-1328) | | | | |

BCM-1979. Temps de doublement de la tumeur = 0.9 jour. Temps moyen pour les tumeurs du groupe excipient pour atteindre 740 mg = 11.9 jours. Formulation : Exemple 2 = 20% labrasol,5% solutol,75% glucose,5% en eau. Abréviations utilisées : BWC= body weight change, HNTD= highest nontoxic dose, HDT = highest dose tested. p.o. = *per os*

EVALUATION DE L'EXEMPLE **19** de W008065282 CONTRE LE MELANOME MURIN PRECOCE **B16**

| Composé (jour d'administration) | Dosage en mg/kg/adm | Dose totale en mg/kg | Mort due au produit (jour de la mort) | %BWC (jour du nadir) | Poids moyen de la tumeur en mg au jour 12 (range) | T/C en % jour 12 | T-C en jours 750 mg | Log de cellule tuée totale | Commentaires |
|---|---|---|---|---|---|---|---|---|---|
| Exemple 19 de W008065282 | 120.0 | 2554.3[b] | 0/7[c] | +8.3(14) | 441 (270-594) | 29 | 4.2 | 1.2 | HDT active |
| p.o. 3-13 (2x/j)[a] | 74.4 | 1588,3[b] | 0/7[c] | +10.6 (14) | 459 (394-949) | 30 | 3,6 | 1,0 | Active |
| | 46.1 | 984.0[b] | 0/7 | -0.0 (4) | 849 (576-1093) | 56 | - | - | Inactive |
| Excipient p.o. 3-13 (2x/j)[a] | - | | 0/10 | +18.3 (14) | 1522 (1000-1762) | | | | |

BCM-1755. Temps de doublement de la tumeur = 1.1 jour. Formulation : Exemple 3 = Captisol 40% en eau, pH3.
Durée du traitement : exemple 3 = 11 jours. Abréviations utilisées : HDT = highest dose tested. [a] Au jour 3, les souris ont été traitées une fois par jour. [b] la dose totale a été réévaluée après le dosage de la solution mère par HPLC les jours 3, 4 et 11 à 13. [c]Une mort accidentelle, efficacité calculée sur 6 animaux.

## Revendications

1. Composé répondant à la formule (I)

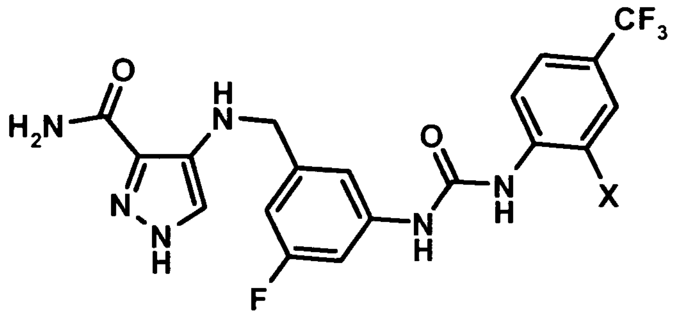

dans laquelle :

X représente chlore ou fluor,
à l'état de base ou de sel d'addition à un acide.

2. Composé de formule (I) selon la revendication 1, **caractérisé en ce que** X représente le chlore, à l'état de base ou de sel d'addition à un acide.

3. Procédé de préparation d'un composé de formule (I) selon l'une quelconque des revendications 1 à 2, **caractérisé en ce que** l'on fait réagir le composé suivant

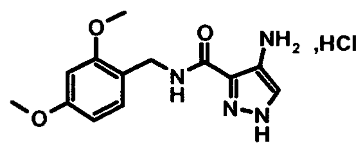

avec le composé

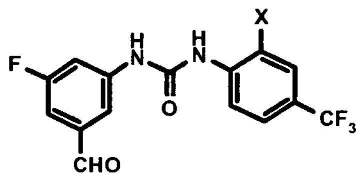

en présence de diisoproplyéthylamine dans un milieu inerte, puis dans une deuxième étape on déprotège l'amine par l'acide para toluène sulfonique.

4. Procédé selon la revendication 3 **caractérisé en ce que** le milieu inerte est un milieu aprotique apolaire.

5. Procédé selon la revendication 4 **caractérisé en ce que** le milieu aprotique apolaire est le tétrahydrofurane.

6. Composé de formule

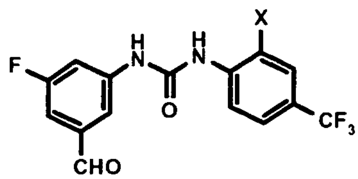

où X représente le chlore ou le fluor.

**7.** Composé de formule

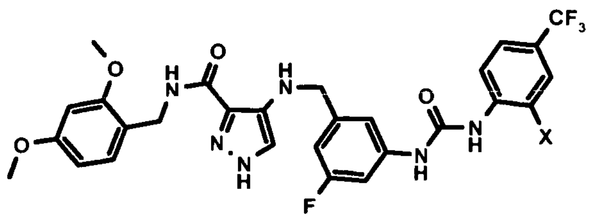

où X représente le chlore ou le fluor.

**8.** Médicament, **caractérisé en ce qu'**il comprend un composé de formule (I) selon l'une quelconque des revendications 1 à 2, ou un sel d'addition de ce composé à un acide pharmaceutiquement acceptable.

**9.** Composition pharmaceutique, **caractérisée en ce qu'**elle comprend un composé de formule (I) selon l'une quelconque des revendications 1 à 2, ou un sel pharmaceutiquement acceptable de ce composé, ainsi qu'au moins un excipient pharmaceutiquement acceptable.

**10.** Composé répondant à la formule (I) selon l'une quelconque des revendications 1 à 2 pour son application comme médicament dans le traitement du cancer.

## Patentansprüche

**1.** Verbindung der Formel (I)

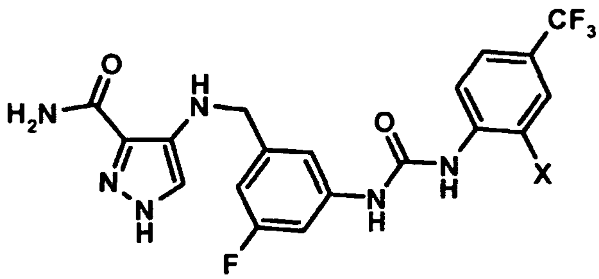

worin:

X für Chlor oder Fluor steht,
in Basen- oder Säureadditionssalzform.

**2.** Verbindung der Formel (I) nach Anspruch 1, **dadurch gekennzeichnet, dass** X für Chlor steht, in Basen- oder Säureadditionssalzform.

**3.** Verfahren zur Herstellung einer Verbindung der Formel (I) nach einem der Ansprüche 1 bis 2, **dadurch gekennzeichnet, dass** man die folgende Verbindung

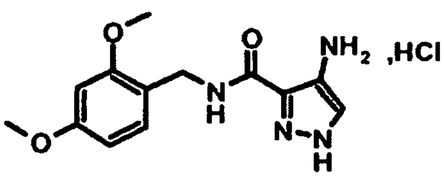

in Gegenwart von Diisopropylethylamin in einem inerten Medium mit der Verbindung

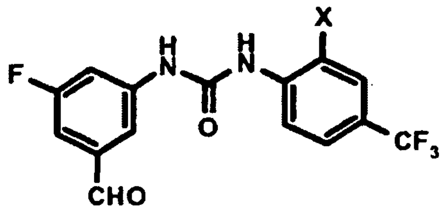

umsetzt und dann in einem zweiten Schritt das Amin mit para-Toluolsulfonsäure entschützt.

4. Verfahren nach Anspruch 3, **dadurch gekennzeichnet, dass** es sich bei dem inerten Medium um ein apolares aprotisches Medium handelt.

5. Verfahren nach Anspruch 4, **dadurch gekennzeichnet** es sich bei dem apolaren aprotischen Medium um Tetrahydrofuran handelt.

6. Verbindung der Formel

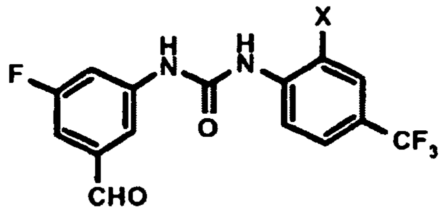

wobei X für Chlor oder Fluor steht.

7. Verbindung der Formel

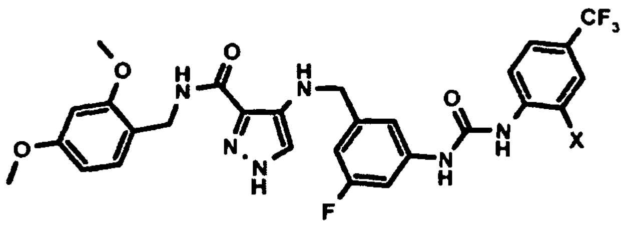

wobei X für Chlor oder Fluor steht.

8. Medikament, **dadurch gekennzeichnet, dass** es eine Verbindung der Formel (I) nach einem der Ansprüche 1 bis 2 oder ein Additionssalz dieser Verbindung mit einer pharmazeutisch unbedenklichen Säure umfasst.

9. Pharmazeutische Zusammensetzung, **dadurch gekennzeichnet, dass** sie eine Verbindung der Formel (I) nach einem der Ansprüche 1 bis 2 oder ein pharmazeutisch unbedenkliches Salz dieser Verbindung sowie mindestens einen pharmazeutisch unbedenklichen Hilfsstoff umfasst.

10. Verbindung der Formel (I) nach einem der Ansprüche 1 bis 2 zur Anwendung als Medikament bei der Behandlung von Krebs.

**Claims**

1. Compound corresponding to the formula (I):

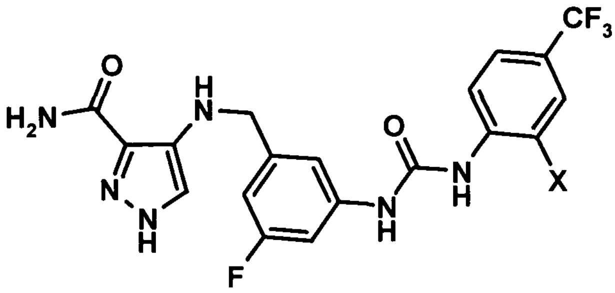

in which:

X represents chlorine or fluorine,
in the form of base or acid-addition salt.

2. Compound of formula (I) according to Claim 1, **characterized in that** X represents chlorine, in the form of base or of acid-addition salt.

3. Process for preparing a compound of formula (I) according to either one of Claims 1 and 2, **characterized in that** the following compound:

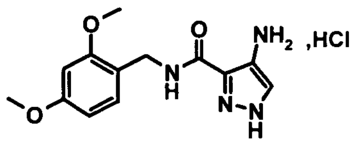

is reacted with the compound:

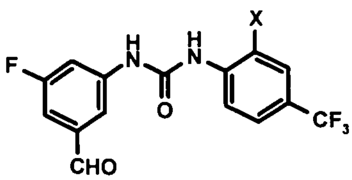

in the presence of diisopropylethylamine in an inert medium, then in a second step, the amine is deprotected by para-toluenesulphonic acid.

4. Process according to Claim 3, **characterized in that** the inert medium is an apolar aprotic medium.

5. Process according to Claim 4, **characterized in that** the apolar aprotic medium is tetrahydrofuran.

6. Compound of formula:

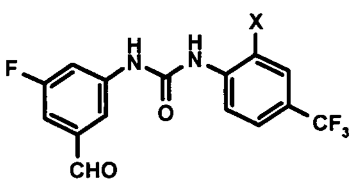

where X represents chlorine or fluorine.

7. Compound of formula:

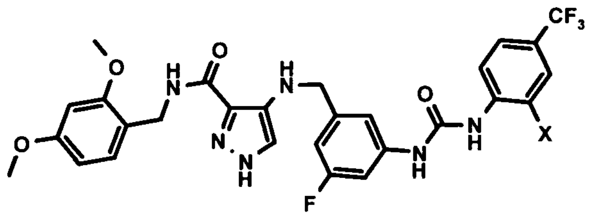

where X represents chlorine or fluorine.

**8.** Medicament, **characterized in that** it comprises a compound of formula (I) according to either one of Claims 1 and 2, or an addition salt of this compound with a pharmaceutically acceptable acid.

**9.** Pharmaceutical composition, **characterized in that** it comprises a compound of formula (I) according to either one of Claims 1 and 2, or a pharmaceutically acceptable salt of this compound, and also at least one pharmaceutically acceptable excipient.

**10.** Compound corresponding to the formula (I) according to either one of Claims 1 and 2 for its application as a medicament in the treatment of cancer.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 08065282 A


### Littérature non-brevet citée dans la description


- **STRAWN et al.** *Cancer Research,* 1996, 3540-3545
- **MILLAUER et al.** *Cancer Research,* 1996, vol. 56, 1615-1620
- **LEE et al.** *Cancer Research,* 2000, vol. 60, 5565-5570
- **S. DAVIS et al.** *Cell,* 1996, vol. 87, 1161-1169
- **P.C. MAISONPIERRE et al.** *Science,* 1997, vol. 277, 55-60
- **ASAHARAT.** *Circ. Res.,* 1998, 233-240
- **D.J. DUMONT et al.** *Genes Dev.,* 1994, vol. 8, 1897-1909
- **C. SURI.** *Cell,* 1996, vol. 87, 1171-1180
- **LIN et al.** *J. Clin. Invest.,* 1997, vol. 100 (8), 2072-2078
- **LIN P.** *PNAS,* 1998, vol. 95, 8829-8834
- *Oncogene,* 19 Mai 2003, vol. 22, 3172-9
- *Nat Med.,* Janvier 1995, vol. 1 (1), 27-31
- *Recent Prog Horm Res.,* 2000, vol. 55 (15-35), 35-6
- *Cell,* 27 Décembre 1996, vol. 87 (7), 1161-9
- *Recent Prog Horm Res.,* 2004, vol. 59, 51-71
- **PENGNIAN LIN ; JAKE A. BUXTON ; ANN ACHESON ; CZESLAW RADZIEJEWSKI ; PETER C. MAISONPIERRE ; GEORGE D. YANCOPOULOS ; KEITH M. CHANNON ; LAURA P. HALE ; MARK W. DEWHIRST ; SAMUEL E. GEORGE.** *Proc Natl Acad Sci USA.,* 21 Juillet 1998, vol. 95 (15), 8829-34
- **CECILIA MELANI ; ANTONELLA STOPPACCIARO ; CHIARA FORONI ; FEDERICA FELICETTI ; ALESSANDRA CARÉ ; MARIOP. COLOMBO.** *Cancer immunol Immunother.,* Juillet 2004, vol. 53 (7), 600-8
- *Cell Signal.,* 14 Avril 2006
- **LAURA M DEBUSK ; DENNIS E HALLAHAN ; PENGNIAN CHARLES LIN.** *Exp Cell Res.,* 01 Août 2004, vol. 298 (1), 167-77
- **TOHRU....MORISADA ; YUICHI OIKE ; YOSHIHIRO YAMADA ; TAKASHI URANO ; MASAKI AKAO ; YOSHIAKI KUBOTA ; HIROMITSU MAEKAWA ; YOSHISHIGE KIMURA ; MASAKO OHMURA ; TAKESHI MIYAMOTO.** *Blood,* 15 Juin 2005, vol. 105 (12), 4649-56
- **SHIBUYA M.** Vascular endothelial growth factor receptor-1 (VEGFR-1/Flt-1): a dual regulator for angiogenesis. *Angiogenesis,* 2007, vol. 9 (4), 225-30
- **TAMELA, T. et al.** Blocking VEGFR-3 suppresses angiogenic sprouting and vascular network formation. *Nature,* 2005, vol. 454, 656-660
- *Br J Cancer.,* 20 Mai 2002, vol. 86 (10), 1566-77
- **THOMAS DA ; GILES FJ ; CORTES J ; ALBITAR M ; KANTARJIAN HM.** *Acta Haematol,* 2001, vol. 207, 106-190
- *Leuk Res.,* Janvier 2006, vol. 30, 54-9
- *Histol Histopathol.,* Octobre 2004, vol. 19 (4), 1245-60
- *Blood,* 15 Juillet 2003, vol. 102 (2), 638-45
- **CORBETT TH.** *Cancer Research,* 1982, vol. 42, 1707-1715
- **T.H. CORBETT.** *Cancer,* 1977, vol. 40, 2660-2680
- **F.M. SCHABEL.** Cancer Drug Development, Part B, Methods in Cancer Research. Academic Press Inc, 1979, vol. 17, 3-51